(19) Europäisches Patentamt
European Patent Office
Office européen des brevets

(11) Veröffentlichungsnummer: **0 022 969**
**B1**

(12) # EUROPÄISCHE PATENTSCHRIFT

(45) Veröffentlichungstag der Patentschrift: **25.05.83**

(21) Anmeldenummer: **80103843.1**

(22) Anmeldetag: **07.07.80**

(51) Int. Cl.³: **A 61 K 31/415,** A 61 K 31/41 // C07D233/60, C07D249/08, A01N43/50, A01N43/64, C07C49/255, C07C49/175

(54) **Antimykotische Mittel, die Azolyl-alkenole enthalten und ihre Herstellung.**

(30) Priorität: **18.07.79 DE 2928968**

(43) Veröffentlichungstag der Anmeldung: **28.01.81 Patentblatt 81/4**

(45) Bekanntmachung des Hinweises auf die Patenterteilung: **25.05.83 Patentblatt 83/21**

(84) Benannte Vertragsstaaten: **BE CH DE FR GB IT LI NL SE**

(56) Entgegenhaltungen:
**EP-A-0 010 674**
**EP-A-0 023 286**
**DE-A-2 350 121**
**DE-A-2 350 122**
**DE-A-2 350 123**
**DE-A-2 350 124**
**GB-A-2 004 276**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Jäger, Gerhard, Dr., Gellertstrasse 18, D-5090 Leverkusen 1 (DE)**
Erfinder: **Kraatz, Udo, Dr., Körner Strasse 6, D-5090 Leverkusen 1 (DE)**
Erfinder: **Büchel, Karl Heinz, Prof. Dr., Haus an der Dachsdelle Dabringhausener Strasse 42, D-5093 Burscheid (DE)**
Erfinder: **Haller, Ingo, Dr., Viktoriastrasse 99, D-5600 Wuppertal 1 (DE)**
Erfinder: **Plempel, Manfred, Dr., Pahlkestrasse 5, D-5600 Wuppertal 1 (DE)**

EP 0 022 969 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Antimykotische Mittel, die Azolyl-alkenole enthalten, und ihre Herstellung.

Die vorliegende Erfindung betrifft antimykotische Mittel, die neue Azolyl-alkenole enthalten.

Es ist bereits bekanntgeworden, daß 1-Ethyl-imidazol- und triazol-Derivate, wie insbesondere 1-(Imidazol-1-yl)- bzw. -(1,2,4-triazol-1-yl)-1-phenoxy-4,4-dimethyl-pentan-3-ole, gute antimykotische Wirkung aufweisen (vergleiche DE-A-2 350 121 bzw. DE-A-2 350 124). Deren Wirkung ist jedoch, insbesondere gegen Dermatophyten, nicht immer ganz befriedigend.

Es wurde gefunden, daß die neuen Azolyl-alkenole der allgemeinen Formel

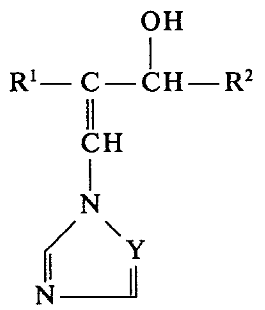

(I)

in welcher

$R^1$ für Phenoxy oder Phenyl steht, wobei die genannten Reste substituiert sein können durch Halogen, Alkyl mit 1 bis 4 Kohlenstoffatomen und durch gegebenenfalls halogen-substituiertes Phenyl,

$R^2$ für Alkyl mit 1 bis 4 Kohlenstoffatomen oder Phenyl steht, wobei letzteres substituiert sein kann durch Halogen, Alkyl mit 1 bis 4 Kohlenstoffatomen und durch gegebenenfalls halogen-substituiertes Phenyl, und

X für ein Stickstoffatom oder die CH-Gruppe steht,

und deren physiologisch verträglichen Säureadditionssalze und Metallsalz-Komplexe gute antimikrobielle, insbesondere antimykotische Eigenschaften aufweisen.

Die genannten Verbindungen als solche sowie diese enthaltende fungizide Mittel sind Gegenstand der eigenen EP-A-0 023 286. Die Verbindungen der Formel (I) können in zwei geometrischen Isomerenformen vorliegen, je nach Anordnung der Gruppen, welche an die Kohlenstoffatome gebunden sind, die durch die Doppelbindung verbunden sind. Die Verbindungen der Formel (I) besitzen ein asymmetrisches Kohlenstoffatom und können deshalb auch in den beiden optischen Isomeren oder als Racemate vorliegen. Sämtliche Isomeren sowie deren Gemische werden erfindungsgemäß verwendet.

Überraschenderweise zeigen die erfindungsgemäß verwendbaren Azolylalkenole der Formel (I) eine bessere antimykotische Wirksamkeit als die aus dem Stand der Technik bekannten 1-(Imidazol-1-yl)- bzw. -(1,2,4-triazol-1-yl)-1-phenoxy-4,4-dimethyl-pentan-3-ole, welche chemisch und wirkungsmäßig naheliegende Verbindungen sind. Die erfindungsgemäß verwendbaren Stoffe stellen somit eine Bereicherung der Pharmazie dar.

Bevorzugt sind diejenigen Verbindungen der Formel (I), in denen $R^1$ für jeweils gegebenenfalls einfach oder mehrfach, gleich oder verschieden durch Fluor, Chlor, Brom, Methyl, Ethyl, Phenyl oder Chlorphenyl substituiertes Phenyl oder Phenoxy steht, $R^2$ für Methyl, Isopropyl, tert.-Butyl, Cyclohexyl, sowie für gegebenenfalls einfach oder mehrfach, gleich oder verschieden durch Fluor, Chlor, Brom, Methyl, Ethyl oder Phenyl substituiertes Phenyl steht, und Y für ein Stickstoffatom oder die CH-Gruppe steht.

Im einzelnen seien außer den bei den Herstellungsbeispielen genannten Verbindungen die folgenden Verbindungen der allgemeinen Formel (I) genannt:

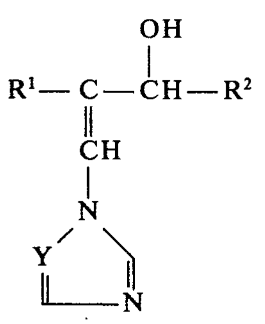

(I)

| $R^1$ | $R^2$ | Y |
|---|---|---|
| 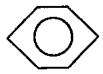 | 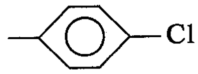 | N |
| 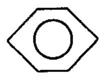 | 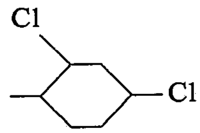 | N oder CH |
| 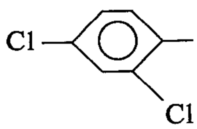 | 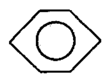 | N oder CH |
| 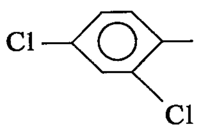 | 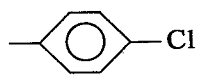 | N oder CH |
| 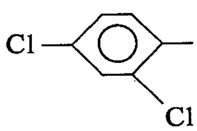 | 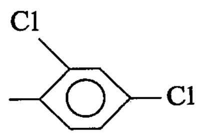 | N oder CH |
| 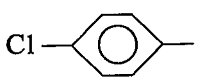 | 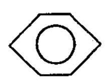 | N oder CH |
| 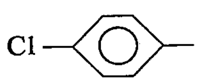 | 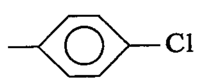 | N oder CH |
| 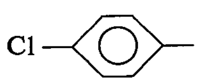 | 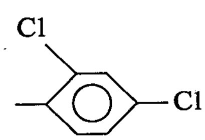 | N oder CH |
| 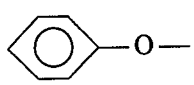 | $C(CH_3)_3$ | N |
| 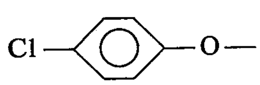 | $C(CH_3)_3$ | N |
| 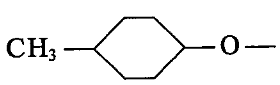 | $C(CH_3)_3$ | N |
| 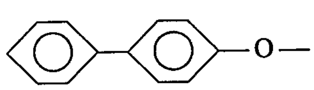 | $C(CH_3)_3$ | N |
| 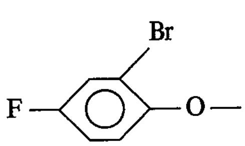 | $C(CH_3)_3$ | N |
| 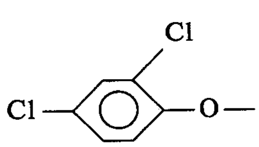 | $C(CH_3)_3$ | N |

Fortsetzung

| $R^1$ | $R^2$ | Y |
|---|---|---|
| 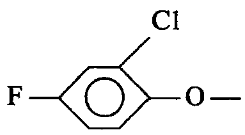 | $C(CH_3)_3$ | N |
| 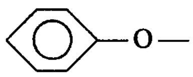 | 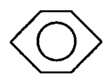 | N oder CH |
| 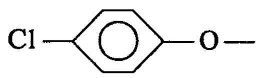 | 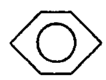 | N oder CH |
| 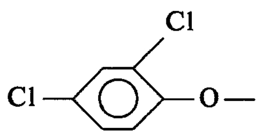 | 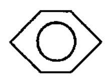 | N oder CH |
| 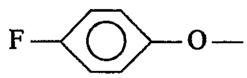 | 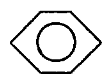 | N oder CH |
| 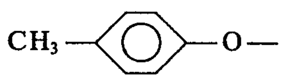 | 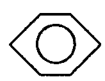 | N oder CH |
| 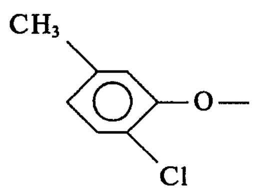 | 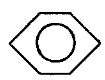 | N oder CH |
| 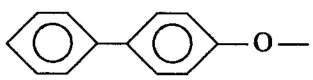 | 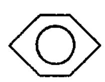 | N oder CH |

Die erfindungsgemäß zu verwendenden Wirkstoffe, deren Säureadditions-Salze und Metallsalz-Komplexe sind noch nicht bekannt. Sie können jedoch gemäß einem eigenen Vorschlag hergestellt werden, indem man Azolyl-alkenone der Formel

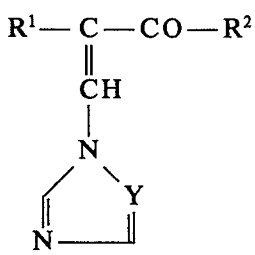

(II)

in welcher

$R^1$, $R^2$ und Y die oben angegebene Bedeutung haben,

nach bekannten Methoden in üblicher Weise reduziert, wie z. B. durch Umsetzung mit komplexen Hydriden oder mit Aluminiumisopropylat.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30° C, vorzugsweise bei 0 bis 20° C durchgeführt. Hierzu setzt

man auf 1 Mol des Ketons der Formel (I) etwa 1 Mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120° C, vorzugsweise bei 50 bis 100° C. Zur Durchführung der Reaktion setzt man auf 1 Mol des Ketons der Formel (I) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Die Azolyl-alkenone der Formel (II) sind Gegenstand einer eigenen älteren Anmeldung (vergleiche EP-A-0 010 674). Die Azolyl-alkenone der Formel (II) werden erhalten, indem man 1-Halogen-ethen-Derivate der Formel

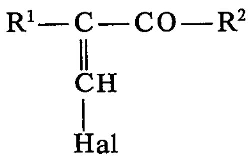 (III)

in welcher

$R^1$ und $R^2$ die oben angegebene Bedeutung haben und
Hal für Halogen, vorzugsweise Chlor oder Brom, steht,

mit Alkalisalzen von Azolen der Formel

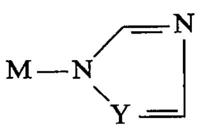 (IV)

in welcher

Y die oben angegebene Bedeutung hat und
M für ein Alkalimetall, vorzugsweise Natrium oder Kalium, steht,

in Gegenwart eines inerten organischen Lösungsmittels, wie beispielsweise Acetonitril, bei Temperaturen zwischen 20 und 120° C umsetzt. Die Isolierung der Verbindungen der Formel (II) erfolgt in üblicher Weise, wobei eine Reinigung gegebenenfalls über ein Säureadditionssalz durchgeführt werden kann (vergleiche auch die Herstellungsbeispiele).

Die Azolyl-alkenone der Formel (II) können auch nach einem eigenen neuen Verfahren erhalten werden, indem man 1-Halogen-ethen-Derivate der Formel

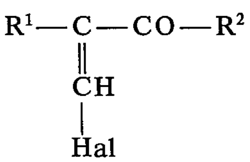 (III)

in welcher

$R^1$, $R^2$ und Hal die oben angegebene Bedeutung haben,

mit Trimethylsilyl-azolen der Formel

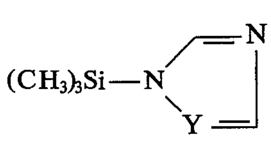 (V)

in welcher

Y die oben angegebene Bedeutung hat,

in Gegenwart eines inerten organischen Verdünnungsmittels, wie beispielsweise Toluol, bei Temperaturen zwischen −10°C bis 120°C umsetzt (vergleiche auch die Herstellungsbeispiele). Die 1-Halogen-ethen-Derivate der Formel (III) sind teilweise bekannt und können in allgemein bekannter Art und Weise erhalten werden, wenn man entsprechende Ethen-Derivate der Formel

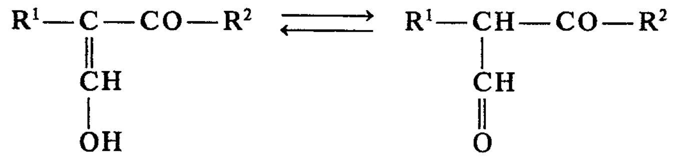

(VI)

in welcher

$R^1$ und $R^2$ die oben angegebene Bedeutung haben,

mit einem Halogenierungsmittel, wie Phosphor- und Schwefelhalogeniden, beispielsweise seien Thionylchlorid, Sulfurylchlorid, Phosphortrichlorid oder -bromid und Phosphoroxychlorid genannt, gegebenenfalls in Gegenwart eines Verdünnungsmittels, wie beispielsweise Toluol oder Xylol, bei Temperaturen zwischen 20 und 100°C umsetzt (vergleiche auch die Herstellungsbeispiele).

Die 1-Hydroxy-ethen-Derivate der Formel (VI) sind teilweise bekannt (vergleiche u. a. Liebigs Ann. Chem. 379, 230 [1911]) bzw. können in allgemein bekannter Art und Weise erhalten werden, indem man bekannte Ketone der Formel

$$R^1-CH_2-CO-R^2 \qquad (VII)$$

in welcher

$R^1$ und $R^2$ die oben angegebene Bedeutung haben,

mit Ameisensäureestern der Formel

$$H-CO-OR^3 \qquad (VIII)$$

in welcher

$R^3$ für Methyl oder Ethyl steht,

in Gegenwart von Natriummethylat bzw. -ethylat in Methanol bzw. Ethanol bei Temperaturen zwischen 0 und 40°C umsetzt (vergleiche auch die Herstellungsbeispiele).

Die Alkalisalze von Azolen der Formel (IV) sind bekannt. Sie werden durch Umsetzung von Imidazol bzw. 1,2,4-Triazol mit Natrium- oder Kaliummethylat in Methanol oder durch Umsetzung von Imidazol mit der äquivalenten Menge des entsprechenden Alkalihydrids erhalten.

Die Trimethylsilyl-azole der Formel (V) sind ebenfalls bekannt (vergleiche DE-OS 1 940 628). Sie werden durch Umsetzung von Imidazol bzw. 1,2,4-Triazol mit Trimethylchlorsilan in Gegenwart einer Base erhalten (vergleiche auch Chem. Ber. 93, 2804).

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z. B. durch Lösen des Metallsalzes in Alkohol, z. B. Äthanol, und

Hinzufügen zur Verbindung der Formel (I). Man kann Metallsalzkomplexe in bekannter Weise, z. B. durch Abfiltrieren, isolieren und gegebenenfalls durch Umkristallisieren reinigen.

Die erfindungsgemäßen Mittel enthalten stets einen oder mehrere pharmazeutisch geeignete Trägerstoffe. Die erfindungsgemäß verwendbaren Verbindungen der Formel (I), ihre Säureadditionssalze und Metallsalzkomplexe weisen starke antimykotische Wirkungen auf, insbesondere gegen Dermatophyten und Sproßpilze sowie bisphasiche Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microspon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabellen, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2,3 oder 4 Einzeldosen oder $^1/_2$, $^1/_3$ oder $^1/_4$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Ricinusöl und

Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Beispiel A

### Antimykotische in-vitro-Wirksamkeit

#### Versuchsbeschreibung:

Die in vitro-Prüfungen wurden im Reihenverdünnungstest mit Keiminokula von durchschnittlich 5 x 10 Keimen/ml Substrat durchgeführt. Als Nährmedium dienten

a) für Dermatophyten und Schimmelpilze:
Sabourand's milieu d'épresive

b) für Hefen:
Isotonic Sensitest-Broth con Oxid.

Die Bebrütungstemperatur betrug 28° C; Bebrütungszeit war 24 Stunden bei Hefen und 96 Stunden bei Dermatophyten und Schimmelpilzen.

Die erfindungsgemäßen Mittel zeigen bei Trichophyton ment. und bei Candida alb. niedrigere MHK-Werte als die bekannten Mittel.

## Beispiel B

### Antimikrobielle in-vivo-Wirksamkeit (oral) bei Mäuse-Candidose

#### Versuchsbeschreibung:

Mäuse vom Typ SPF-$CF_1$ wurden intravenös mit $1-2 \times 10^6$ logarithmisch wachsenden Candida-Zellen, die in Physiologischer Kochsalzlösung suspendiert waren, infiziert. Eine Stunde vor und sieben Stunden nach der Infektion werden die Tiere mit jeweils 50—100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5%.

Dagegen zeigten z. B. die erfindungsgemäßen Verbindungen der Beispiele 1, 2, 3, 4 und 9 eine gute bis sehr gute Wirkung (≥80% bis 95% Überlebenden am 6. Tag p. i.).

Beispiel C

Antimikrobielle in-vivo-Wirksamkeit (lokal) am Modell der experimentellen Meerschweinchen-Trichophytie

Versuchsbeschreibung:

Weiße Meerschweinchen der Rasse Pirbright-white wurden auf dem geschorenen, nicht skarifizierten Rücken mit einer Mikro- und Makrokonidien-Suspension von Trichophyton mentagrophytes infiziert.

Die infizierten Tiere wurden, beginnend mit dem 3. Tag p. i., 1 × täglich mit 1%iger Lösung der erfindungsgemäßen Präparate (in Dimethylsulfoxid : Glycerin = 1 : 4) lokal behandelt.

Ergebnis:

Bei unbehandelten Tieren entwickelt sich innerhalb von 12 Tagen p. i. das typische Bild einer Dermatophytose mit Rötung, Schuppung und Haarausfall bis zum totalen Integument-Defekt an der Infektionsstelle.

Dagegen zeigten z. B. die erfindungsgemäßen Verbindungen der Beispiele 1, 5 und 9 eine gute bis sehr gute Wirkung, d. h. lediglich geringe Rötung und vereinzelt Schuppen bzw. keine Infektionszeichen.

Herstellungsbeispiele

Beispiel 1

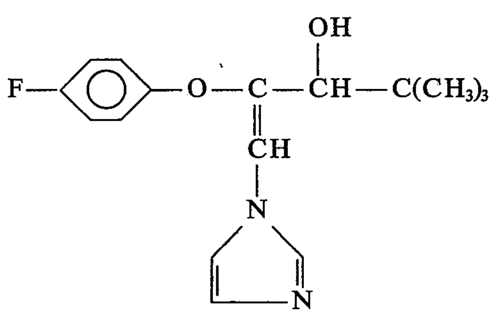

In eine Lösung von 22 g (0,076) Mol) 1-(Imidazol-1-yl)-2-(4-fluorphenoxy)-4,4-dimethyl-1-penten-3-on in 200 ml Methanol werden unter leichter Außenkühlung bei 20—30° C portionsweise 2,9 g (0,076 Mol) Natriumboranat innerhalb von 20 Minuten unter Rühren eingetragen. Nach 3 Stunden wird das Reaktionsgemisch mit 10%iger Essigsäure auf pH 5 gestellt und anschließend unter vermindertem Druck eingedampft.

Der verbleibende ölige Rückstand wird in 250 ml Essigester aufgenommen und zweimal mit je 50 ml Wasser ausgeschüttelt. Nach dem Trocknen der organischen Phase über wasserfreiem Natriumsulfat wird die Lösung im Vakuum eingedampft, der verbleibende Rückstand in sehr wenig Ether gelöst und langsam mit Petrolether versetzt. Die ausgeschiedenen farblosen Kristalle werden filtriert mit wenig Petrolether gewaschen und bei 100° C getrocknet. Man erhält 13,7 g (62% der Theorie) 1-(Imidazol-1-yl)-2-(4-fluorphenoxy-4,4-dimethyl-1-penten-3-ol vom Schmelzpunkt 112—113° C.

Herstellung des Ausgangsproduktes

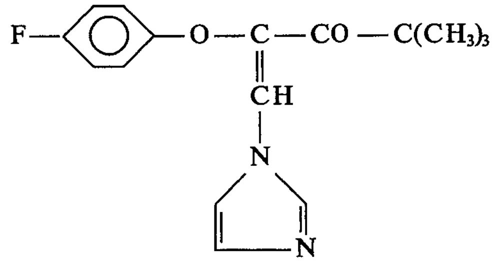

Zu einer Suspension von 90 g (1 Mol) Natrium-imidazol, hergestellt aus Natriummethylat und Imidazol in Methanol, in 2500 ml Acetonitril werden 256,7 g (1 Mol) 1-Chlor-2-(4-fluorphenoxy)-4,4-dimethyl-1-penten-3-on in 150 ml Acetonitril unter Rühren getropft. Danach wird das Reaktionsgemisch 6 Stunden zum Sieden erhitzt. Man läßt auf Raumtemperatur abkühlen und engt durch Abdestillieren des Lösungsmittels im Vakuum ein. Der Rückstand wird mit 1000 ml Essigester aufgenommen, dreimal mit je 200 ml Wasser gewaschen, die organische Phase über Natriumsulfat getrocknet und durch Abdestillieren des Lösungsmittels im Vakuum eingeeengt. Man erhält 272,4 g (94,5% der Theorie) rohes 1-(Imidazol-1-yl)-2-(4-fluorphenoxy)-4,4-dimethyl-1-penten-3-on als braunes Öl, das in üblicher Weise über das Nitrat gereinigt wird und dann einen Brechungsindex $n_D^{20} = 1,5590$ besitzt.

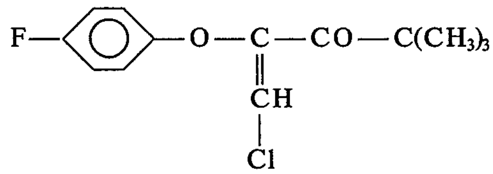

297,5 g (2,5 Mol) Thionylchlorid werden langsam in eine auf 60° C erwärmte Lösung von 404,6 g (1,7 Mol) 1-Hydroxy-2-(4-fluorphenoxy)-4,4-dimethyl-1-penten-3-on in 3000 ml wasserfreiem Toluol eingerührt. Man hält 12 Stunden bei dieser Temperatur und destilliert anschließend das Lösungsmittel und überschüssiges Thionylchlorid ab. Das zurückbleibende Öl wird im Vakuum destilliert. Man erhält 353,3 g (81% der Theorie) 1-Chlor-2-(4-fluorphenoxy)-4,4-dimethyl-1-penten-3-on vom Siedepunkt 95—103° C/0,3 mm.

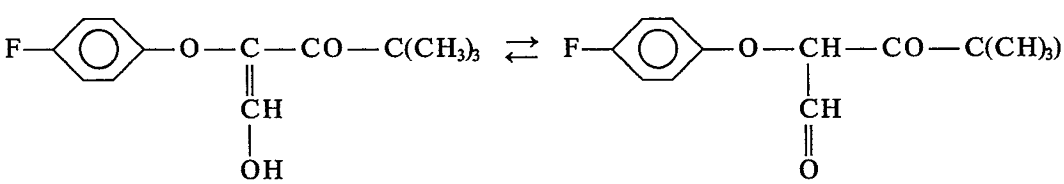

163 g (2,2 Mol) Ameisensäureethylester werden bei 0° C zu einer Lösung von 136 g Natriumethylat in 1500 ml Ethanol getropft. Anschließend werden bei 0° C 420 g (2 Mol) 2,2-Dimethyl-4-(4-fluorphenoxy)-butan-3-on langsam eingerührt. Nach 24stündiger Reaktionszeit bei 0° C läßt man auf Raumtemperatur erwärmen und rührt bei dieser Temperatur noch 96 Stunden nach. Das Reaktionsgemisch wird auf 5000 ml Eiswasser gegeben und die organische Phase wird durch Extrahieren mit Chloroform abgetrennt. Aus dieser Chloroformlösung kann nichtumgesetztes Ausgangsprodukt isoliert und erneut eingesetzt werden. Die wäßrige Phase wird unter Kühlung mit 10%iger Salzsäure angesäuert und das sich ausscheidende Öl in Chloroform aufgenommen. Die Chloroformphase wird über Natriumsulfat getrocknet und durch Abdestillieren des Lösungsmittels im Vakuum eingeengt. Das zurückbleibende Öl wird im Vakuum destilliert. Man erhält 170 g (83% der Theorie, bezogen auf umgesetztes Produkt) 1-Hydroxy-2-(4-fluorphenoxy)-4,4-dimethyl-1-penten-3-on vom Siedepunkt 101—102° C/0,6 mm ($n_D^{20} = 1,5132$).

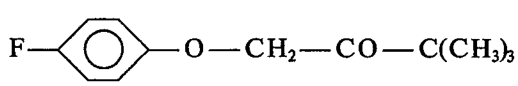

418,3 g (3,11 Mol) 2,2-Dimethyl-4-chlor-butan-3-on werden in eine zum Sieden erhitzte Suspension von 315 g (2,8 Mol) 4-Fluor-phenol und 386,4 g (2,8 Mol) Kaliumcarbonat in 1500 ml Aceton getropft. Man läßt 4 Stunden unter Rückfluß rühren. Nach Abkühlung auf Raumtemperatur wird das

ausgeschiedene Salz abfiltriert und das Filtrat im Vakuum eingeengt. Das zurückbleibende Öl wird im Vakuum destilliert. Man erhält 101,5 g (86,2% der Theorie) 2,2-Dimethyl-4-(4-fluorphenoxy)-butan-3-on vom Siedepunkt 83—84° C/0,05 mm ($n_D^{20}$ = 1,4919).

Beispiel 2

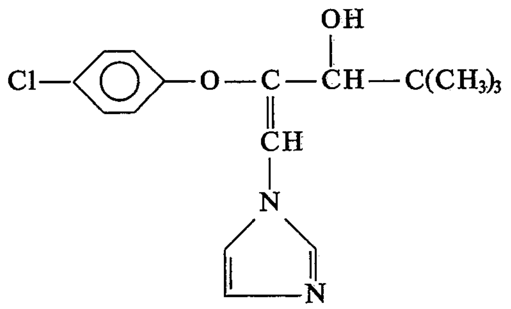

In eine Lösung von 98,8 g (0,33 Mol) 1-(Imidazol-1-yl)-2-(4-Chlorphenoxy)-4,4-dimethyl-1-penten-3-on in 1000 ml Methanol werden unter leichter Außenkühlung bei 20—30° C portionsweise 12,4 g (0,33 Mol) Natriumboranat innerhalb von 30 Minuten eingetragen. Nach 3 Stunden wird die Lösung filtriert, das Filtrat mit 10%iger Essigsäure angesäuert und unter vermindertem Druck eingedampft. Der verbleibende feste Rückstand wird in 1,3 Ltr. Wasser eingerührt. Das wasserunlösliche Reduktionsprodukt wird abfiltriert und im Vakuum bei 50° C getrocknet.

Man erhält 93,7 g (92,55% der Theorie) 1-(Imidazol-1-yl)-2-(4-chlorphenoxy)-4,4-dimethyl-1-penten-3-ol vom Schmelzpunkt 141—143° C in Form farbloser Kristalle.

Herstellung des Ausgangsproduktes

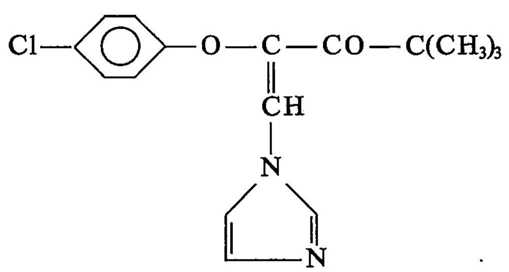

(Neue Verfahrensvariante)

13 g (0,05 Mol) 1-Chlor-2-(4-chlorphenoxy)-4,4-dimethyl-1-penten-3-on und 7 g (0,05 Mol) Trimethylsilylimidazol werden in 50 ml Toluol 5 Stunden unter Rückfluß erhitzt. Danach wird das Reaktionsgemisch durch Andestillieren des Lösungsmittels im Vakuum eingeengt und der ölige Rückstand säulenchromatographisch getrennt (Kieselgel; Essigester : Chloroform = 1 : 2). Man erhält 9 g (58% der Theorie) 1-Imidazol-1-yl)-2-(4-chlorphenoxy)-4,4-dimethyl-1-penten-3-on vom Schmelzpunkt 94—95° C.

In entsprechender Weise werden die nachfolgenden Verbindungen der allgemeinen Formel:

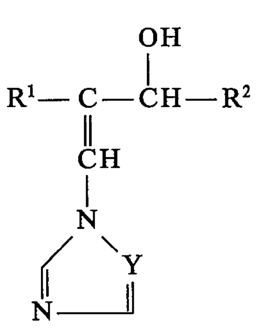

(I)

erhalten:

| Beispiel Nr. | $R^1$ | $R^2$ | Y | Schmelzpunkt (°C) |
|---|---|---|---|---|
| 3 | 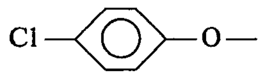 | $C(CH_3)_3$ | CH | 195–97 (xHCl) |
| 4 | 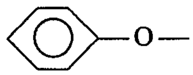 | $C(CH_3)_3$ | CH | 133–34 |
| 5 | 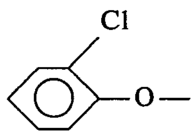 | $C(CH_3)_3$ | CH | 103–05 |
| 6 | 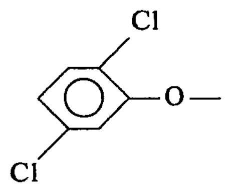 | $C(CH_3)_3$ | CH | 110–22 |
| 7 | 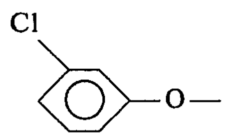 | $C(CH_3)_3$ | CH | 107–08 |
| 8 | 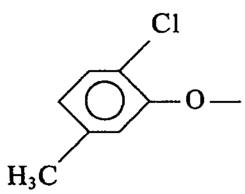 | $C(CH_3)_3$ | CH | 137–38 |
| 9 | 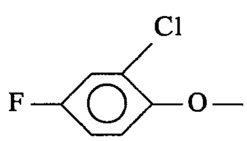 | $C(CH_3)_3$ | CH | 75–88 |
| 10 | 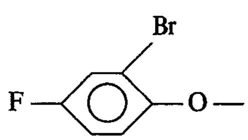 | $C(CH_3)_3$ | CH | 75–93 |
| 11 | 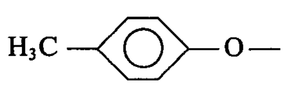 | $C(CH_3)_3$ | CH | 140–41 |
| 12 | 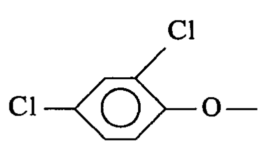 | $C(CH_3)_3$ | CH | 106–08 (Zers.) |
| 13 | 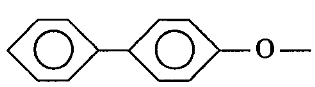 | $C(CH_3)_3$ | CH | 80–82 (Zers.) |
| 14 | 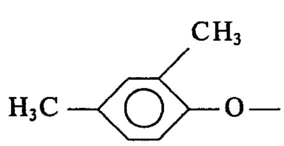 | $C(CH_3)_3$ | CH | 110–14 |

Fortsetzung

| Beispiel Nr. | $R^1$ | $R^2$ | Y | Schmelzpunkt (°C) |
|---|---|---|---|---|
| 15 | 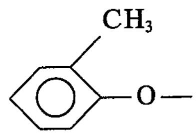 | $C(CH_3)_3$ | CH | 92–95 |
| 16 | 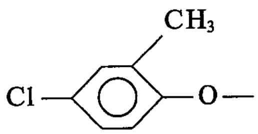 | $C(CH_3)_3$ | CH | 83–85 |
| 17 | 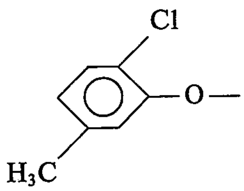 | $C(CH_3)_3$ | N | 150–53 |
| 18 | 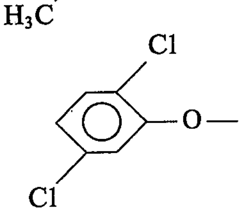 | $C(CH_3)_3$ | N | 136–37 |
| 19 | 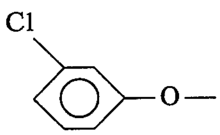 | $C(CH_3)_3$ | N | 98–99 |
| 20 | 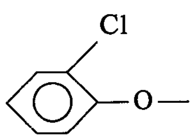 | $C(CH_3)_3$ | N | 75–80 |
| 21 | 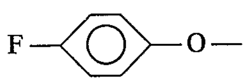 | $C(CH_3)_3$ | N | 118–19 |
| 22 | 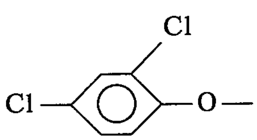 | $C(CH_3)_3$ | N | 125–30 |
| 23 | 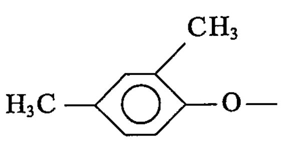 | $C(CH_3)_3$ | N | 118–19 |
| 24 | 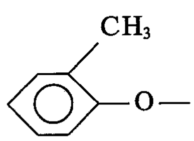 | $C(CH_3)_3$ | N | 73–78 |
| 25 | 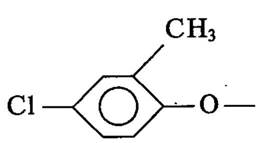 | $C(CH_3)_3$ | N | 117–18 |

Fortsetzung

| Beispiel Nr. | $R^1$ | $R^2$ | Y | Schmelzpunkt (°C) |
|---|---|---|---|---|
| 26 | 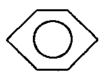 | 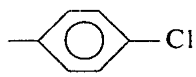 | N | 116–18 |
| 27 | 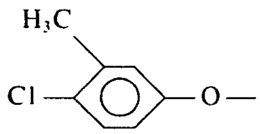 | $C(CH_3)_3$ | CH | 148–49 |
| 28 | 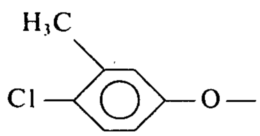 | $C(CH_3)_3$ | N | 108–09 |

**Patentansprüche**

1. Antimykotische Mittel, gekennzeichnet durch einen Gehalt an mindestens einem Azolyl-alkenol der allgemeinen Formel

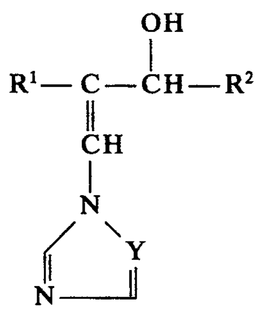

(I)

in welcher

$R^1$ für Phenoxy oder Phenyl steht, wobei die genannten Reste substituiert sein können durch Halogen, Alkyl mit 1 bis 4 Kohlenstoffatomen und durch gegebenenfalls halogen-substituiertes Phenyl,
$R^2$ für Alkyl mit 1 bis 4 Kohlenstoffatomen oder Phenyl steht, wobei letzteres substituiert sein kann durch Halogen, Alkyl mit 1 bis 4 Kohlenstoffatomen und durch gegebenenfalls halogen-substituiertes Phenyl, und
Y für ein Stickstoffatom oder die CH-Gruppe steht,

und/oder deren physiologisch verträglichen Säureadditions-Salze und Metallsalz-Komplexe und einem pharmazeutisch geeigneten Trägerstoff.

2. Antimykotisches Mittel gemäß Anspruch 1, gekennzeichnet durch einen Gehalt an 1-(Imidazol-1-yl)-2-(4-fluorphenoxy)-4,4-dimethyl-1-penten-3-ol.

3. Antimykostisches Mittel gemäß Anspruch 1, gekennzeichnet durch einen Gehalt an 1-(Imidazol-1- l)-2-(4-chlorphenoxy)-4,4-dimethyl-1-penten-3-ol.

4. Antimykotisches Mittel gemäß Anspruch 1, gekennzeichnet durch einen Gehalt an 1-(Imidazol-1-yl)-2-(4-chlorphenoxy)-4,4-dimethyl-1-penten-3-ol-hydrochlorid.

5. Antimykotisches Mittel gemäß Anspruch 1, gekennzeichnet durch einen Gehalt an 1-(Imidazol-1- l)-2-phenoxy-4,4-dimethyl-1-penten-3-ol.

6. Antimykotisches Mittel gemäß Anspruch 1, gekennzeichnet durch einen Gehalt an 1-(Imidazol-1-yl)-2-(2-chlor-4-fluorphenoxy)-4,4-dimethyl-1-penten-3-ol.

7. Verfahren zur Herstellung eines antimykotischen Mittels, dadurch gekennzeichnet, daß man Azolylalkenole gemäß der Formel in Anspruch 1 mit inerten, nichttoxischen, pharmazeutisch geeigneten Trägerstoffen vermischt.

## Claims

1. Antimycotic agents, characterized in that they contain at least one azolyl-alkenol of the general formula

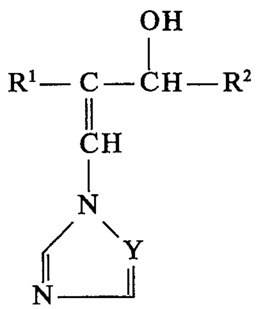

(I)

in which

$R^1$ represents phenoxy or phenyl, it being possible for the stated radicals to be substituted by halogen, alkyl with 1 to 4 carbon atoms or by optionally halogen-substituted phenyl,
$R^2$ represents alkyl with 1 to 4 carbon atoms or phenyl, it being possible for the latter to be substituted by halogen, alkyl with 1 to 4 carbon atoms or by optionally halogen-substituted phenyl, and
Y represents a nitrogen atom or the CH group,

and/or physiologically acceptable acid addition salts and metal salt complexes thereof and a pharmaceutically suitable excipient.

2. Antimycotic agent according to claim 1, characterized in that it contains 1-(imidazol-1-yl)-2-(4-fluorophenoxy)-4,4-dimethyl-1-penten-3-ol.

3. Antimycotic agent according to claim 1, characterized in that it contains 1-(imidazol-1-yl)-2-(4-chlorophenoxy)-4,4-dimethyl-1-penten-3-ol.

4. Antimycotic agent according to claim 1, characterized in that it contains 1-(imidazol-1-yl)-2-(4-chlorophenoxy)-4,4-dimethyl-1-penten-3-ol hydrochloride.

5. Antimycotic agent according to claim 1, characterized in that it contains 1-(imidazol-1-yl)-2-phenoxy-4,4-dimethyl-1-penten-3-ol.

6. Antimycotic agent according to claim 1, characteriszed in that it contains 1-(imidazol-1-yl)-2-(2-chloro-4-fluorophenoxy)-4,4-dimethyl-1-penten-3-ol.

7. Process for the preparation of an antimycotic agent, characterized in that azolyl-alkenols according to the formula in claim 1 are mixed with inert, non-toxic, pharmaceutically suitable excipients.

## Revendications

1. Agents antimycotiques, caractérisés en ce qu'ils contiennent au moins un azolyl-alcénol de formule générale

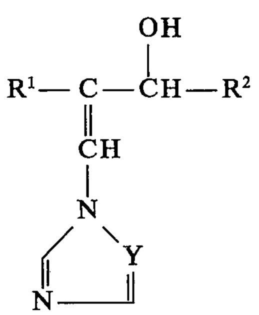

(I)

dans laquelle

$R^1$ représente un groupe phénoxy ou phényle, les restes mentionnés pouvant être substitués par un reste halogène, alkyle en $C_1-C_4$ et par phényle éventuellement halogéno-substitué,
$R^2$ représente un groupe alkyle en $C_1-C_4$ ou phényle, ce dernier pouvant être substitué par un reste halogène ou alkyle en $C_1-C_4$ et par un reste phényle éventuellement halogéno-substitué, et
Y représente un atome d'azote ou le groupe CH,

et/ou leurs sels d'addition d'acides et complexes de sels métalliques physiologiquement acceptables et un support approprié en pharmacie.

2. Agent antimycotique selon la revendication 1, caractérisé en ce qu'il contient du 1-(imidazole-1-yl)-2-(4-fluoro-phénoxy)-4,4-diméthyl-1-pentène-3-ol.

3. Agent antimycotique selon la revendication 1, caractérisé en ce qu'il contient du 1-(imidazole-1-yl)-2-(4-chlorophénoxy)-4,4-diméthyl-1-pentène-3-ol.

4. Agent antimycotique selon la revendication 1, caractérisé en ce qu'il contient du chlorhydrate de 1-(imidazole-1-yl)-2-(4-chlorophénoxy)-4,4-diméthyl-1-pentène-3-ol.

5. Agent antimycotique selon la revendication 1, caractérisé en ce qu'il contient du 1-(imidazole-1-yl)-2-phénoxy-4,4-diméthyl-1-pentène-3-ol.

6. Agent antimycotique selon la revendication 1, caractérisé en ce qu'il contient du 1-(imidazole-1-yl)-2-(2-chloro-4-fluoro-phénoxy)-4,4-diméthyl-1-pentène-3-ol.

7. Procédé pour la fabrication d'un agent antimycotique, caractérisé en ce que l'on mélange des azolyl-alcénols selon la formule dans la revendication 1 avec des supports inertes non toxiques appropriés en pharmacie.